Europäisches Patentamt

(19) European Patent Office

Office européen des brevets

(11) Publication number: **0 136 798**
**A2**

(12) # EUROPEAN PATENT APPLICATION

(21) Application number: **84305697.9**

(22) Date of filing: **21.08.84**

(51) Int. Cl.⁴: **G 01 N 33/569**
**G 01 N 33/545**

(30) Priority: **25.08.83 US 526573**
**04.05.84 US 607066**

(43) Date of publication of application:
**10.04.85 Bulletin 85/15**

(84) Designated Contracting States:
**BE DE FR GB IT LU NL SE**

(71) Applicant: **BIOTECH RESEARCH LABORATORIES INC.**
**1600 East Gude Drive**
**Rockville Maryland 20850(US)**

(72) Inventor: **Bodner, Anne**
**8304 Fenway Road**
**Bethesda Maryland 20817(US)**

(72) Inventor: **Ting, Robert C.Y.**
**9604 Persimmon Tree Road**
**Potomac Maryland 20854(US)**

(74) Representative: **Collier, Jeremy Austin Grey et al,**
**J.A.Kemp & Co. 14, South Square Gray's Inn**
**London WC1R 5EU(GB)**

(54) **Titre plate and assay kit for detection of antibodies in human serum and their production and use.**

(57) An assay kit for the quantitative and qualitative detection of antibodies to the family of Human T-Cell Leukemia Viruses (HTLV) in humans, comprises a novel HTLV antigen-coated microtiter plate and lyophilized reagents. This assay kit has a shelf-life of greater than about six months.

EP 0 136 798 A2

## TITRE PLATE AND ASSAY KIT FOR DETECTION OF ANTIBODIES
## IN HUMAN SERUM AND THEIR PRODUCTION AND USE

### FIELD OF THE INVENTION

This invention relates generally to the field of immunology, and more specifically, it relates to a method of producing a sensitive assay kit of long range stability for the detection of antibodies in human serum.

### BACKGROUND OF THE INVENTION

Recent scientific advancements have linked the Human T-cell leukemia-lymphoma virus (HTLV) to several adult Human T-cell malignancies. See, for example, Proc. Nat'l Acad. Sci. U.S.A., vol. 77, <u>Detection and isolation of type C retrovirus particles from fresh and cultured lymphocytes of a patient with cutaneous T-cell lymphoma</u>, Poiesz <u>et al</u>., December, 1980 and Vol. 79, <u>Isolation and characterisation of retrovirus from cell lines of human adult T-cell leukemia and its implication in the disease,</u> Yoshida <u>et al</u>., March, 1982. At present, both HTLV and T-cell malignancies are believed to occur at increased rates in the populations of the Carribean islands and southern Japan, although HTLV has been found in some individuals with lymphoid malignancies in this country. See, for example, Proc. Nat'l Acad. Sci. U.S.A., Vol. 78, Adult T-cell leukemia: Antigen in an ATL cell line and detection of antibodies to the antigen in human sera, Hinuma et al., October, 1981; Science, Vol. 215, Natural Antibodies to Human Retrovirus HTLV in a Cluster of Japanese Patients with Adult T Cell Leukemia, Robert-Guroff et al, February, 1982; Int. J. Cancer, Vol. 30, The Human Type-C Retrovirus, HTLV, in Blacks From the Caribbean Region, and Relationship to Adult T-Cell Leukemia/Lympoma, Blattner et al., 1982. In addition, the HTLV virus has taken on new significance with the recent discovery of antibodies to HTLV in patients suffering from acquired immune deficiency syndrome (AIDS). See, for example, Science, Vol. 220, Antibodies to Cell Membrane Antigens Associated with Human T-Cell Leukemia Virus in Patients with AIDS, Essex et al., May, 1983. Continuing research has shown that there are at least three strains of the HTLV virus, presently identified as HTLV-I, HTLV-II, and HTLV-III. HTLV-I is obtained primarily from patients with mature T-cell malignancies. HTLV-II has been isolated from a patient with a T-cell variant of hairy cell leukemia. Science, Vol. 220, Isolation of Human T-Cell Leukemia Virus in Acquired Immune Deficiency Syndrome (AIDS), Gallo et. al, 1983. Although both HTLV-I and HTLV-II can sometimes be found in patients with AIDS, current studies indicate that the primary cause of AIDS may be the HTLV-III strain. Science, Vol. 224, Antibodies Reactive with Human T-Lymphotropic Retroviruses (HTLV-III) in the Serum of Patients with AIDS, Sarngadharan et al, April, 1983; Science, Vol. 224, Detection, Isolation and Continuous Production of Cytopathic Retroviruses (HTLV-III) from Patients with AIDS and Pre-AIDS, Popovic et al, April, 1984; Science, Vol. 224, Frequent Detection and Isolation of Cytopathic Retroviruses (HTLV-III) from Patients with AIDS and at Risk for AIDS, Gallo et al, April, 1984; Science, Vol. 224, Serological Analysis of a Subgroup of Human I-Lymphotropic Retroviruses (HTLV-III) Associated with AIDS, Schupbach et al, April, 1984. Thus, the importance of developing a rapid, accurate, and inexpensive method of screening for antibodies to HTLV antigens, such as HTLV-I, HTLV-II, and especially HTLV-III antigens in human sera, is readily apparent. It is to be understood that the term "HTLV antigen" refers to at least the three known strains of HTLV, i.e., HTLV-I, HTLV-II, and HTLV-III, and any other strain which may be found to fall within the family of HTLV viruses.

The presence of a virus such as HTLV I, HTLV-II or HTLV-III is often detected by testing for the presence of antibodies to HTLV in the patient's serum. Because a disease such as AIDS is highly infectious when transmitted through blood transfusions, as well as other means, a quick, simple and accurate test is needed to screen the thousands of blood samples taken each day by hospitals and blood banks across the country. Although many procedures have been developed to survey serum samples for evidence of exposure to HTLV antigens, such as radioimmunoassay and immunoflourescence, the wide-spread use of most of these techniques is limited by the time and/or expense required to perform them. However, it has been found that the enzyme-linked immunosorbent assay (ELISA) has the sensitivity and specificity of other procedures, yet is highly adaptable to mass screening in automation and requires little or no expensive equipment.

The ELISA assay is based on the use of a covalently linked enzyme-antibody conjugate in which the catalytic and immunologic activities are preserved. The enzymes used are stable, unlike radioactive labels, and are cheaper and simpler to use.

To estimate the level of serum antibodies using the ELISA assay, the corresponding antigens, such as HTLV-I, -II or -III antigens are adsorbed to a plastic microtiter plate. The adsorbed antigens are used to bind specific antibodies which may be present in the test serum. The specifically bound antibodies are then measured by an enzyme-antibody conjugate whose antibody is specific for the immunoglobulins (Igs) which are present in the test serum.

Until the present invention, the microtiter plates and reagents used to perform the ELISA assay for the detection of antibodies to HTLV antigens had to be prepared in the research laboratory only as needed because the microtiter plates and reagents could only be stored for a maximum of one to two weeks before deteriorating. Thus, researchers spent a great deal of time merely preparing the microtiter plates and reagents prior to testing. Furthermore, such short-term stability rendered commercial mass production of the HTLV antigen-coated plates for shipment elsewhere impossible. Also, until the present invention, researchers utilized a wet method of preparing microtiter plates for detection of antibodies to HTLV antigens. The wet plates produced by this method are totally unsuitable for shipping long distances. Since it will be necessary to run thousands of assays a day to screen for HTLV-III antibodies in virtually every blood bank and hospital in the country, an assay kit which is stable for long periods of time is essential to the safety and health of all recipients of donated blood. The assay kit and method of the present invention also provide an inexpensive and accurate test which now can be made available to doctors and hospitals for widespread use to assist in the diagnosis of AIDS.

The present invention provides a novel method of producing an antigen-coated microtiter plate for the qualitative and quantitative detection of antibodies in human serum. The invention also provides a complete assay kit for the detection of antibodies in human serum, including a microtiter plate and lyophilized reagents. This assay kit may be used, in particular, for the detection of antibodies to HTLV-I, HTLV-II and HTLV-III antigens in human serum and remains stable for periods of at least six months.

According to the present invention an HTLV antigen-coated titer plate is produced by a method which comprises: a) adding an aliquot of a diluted lot of HTLV antigens to the wells of a multi-well titer plate; b) storing said titer plate for sufficient time to permit a maximum amount of said HTLV antigens to adsorb onto said titer plate; c) removing any unadsorbed HTLV antigens from said titer plate; d) stabilizing said adsorbed HTLV antigens onto said plate; and e) storing said titer plate in a moisture-free container until ready to seal said plate.

The assay kit according to the present invention includes at least one multi-well microtiter plate on to which antigens have been adsorbed and fixed, preferably by the aforesaid method, one vial of lyophilized human serum containing known levels of antibodies for use as a positive control, one vial of lyophilized human serum which does not contain antibodies for use as a negative control, and one vial of lyophilized enzyme-antibody conjugate for measuring the level of antibodies in a test sample of human serum. Although only the use of a multi-well titer plate is described in the assay kit of the present invention, it is understood that any carrier suitable for adsorbing HTLV antigens, such as beans or tubes, can be substituted for the multi-well titer plates of the present invention.

## DESCRIPTION OF THE PREFERRED EMBODIMENTS

(1)  Method of Preparing And Diluting HTLV Antigens

In the present invention, HTLV-I, HTLV-II and HTLV-III antigens are derived from HTLV viral particles produced by any one of several cell lines, e.g., HUT-102, MT-2, SGO-HTLV-II and HTLV-IIIA.  See, for example, Proc. Nat'l Acad. Sci. U.S.A., Vol. 77, Detection and isolation of type C retrovirus particles from fresh and cultured lymphocytes of a patient with cutaneous T-cell lymphoma, Poiesz et al., December, 1980; Science, Vol. 217, Transformation of Human Leukocytes by Cocultivation with an Adult T Cell Leukemia Virus Producer Cell Line, Yamamoto, et al., August, 1982.  However, it is understood that the HTLV-I, HTLV-II and HTLV-III antigens may be obtained from synthetically produced virus antigens or parts of virus antigens or from virus antigens which are produced using recombinant DNA techniques.

The HTLV viral particles are isolated using standard techniques.  For example, the media supernatant of a culture of an HTLV infected cell-line is gathered and concentrated until the viral particles are at a desired concentration.  The viral particles are then isolated by sucrose gradient ultra-centrifugation.  The isolated viral particles are centrifuged and the viral particles are disrupted by resuspension in a detergent such as Nonidet P40 or Triton X-100.  The HTLV antigens can be used directly at this point or the HTLV antigens can be further purified by known techniques to remove any unwanted proteins which could cause undesirable nonspecific reactions with the sample test serum.

- 7 -

The optimum dilution of each lot of purified HTLV antigen concentrate is determined by standard titration procedures. For instance, two-fold dilutions of sample aliquots of each lot of HTLV antigens are made, ranging from 1:1000 to 1:64,000-fold. The dilutions are then tested by the ELISA assay against samples of human serum which have known positive or negative levels of antibodies to HTLV antigens and also against dilutions of monoclonal antibodies to HTLV antigens.

The dilution of HTLV antigen concentrate is chosen which yields the desired optical density readings (OD) for samples containing known levels of antibodies to HTLV antigens.

(2)   Method of Producing An HTLV Antigen-Coated Microtiter Plate

Upon determining the optimum dilution of HTLV antigen concentrate, an aliquot of diluted HTLV antigen concentrate is added to each well of a plastic, usually polystyrene, multi-well microtiter plate. Examples of suitable plates are the Immulon 1 microtiter plate, Cat. No. 78-951-99, manufactured by Dynatech Laboratories, Inc., 900 Slaters Lane, Alexandria, Va. and the Titertech microtiter plate manufactured by Flow Laboratories, Inc., McClean, Va. The plate is allowed to stand for sufficient time to permit a maximum amount of HTLV antigens to adsorb to the plate. Each well of the microtiter plate is rinsed with deionized water to remove any excess HTLV antigens, and the plate is allowed to dry. In an alternative method of producing an HTLV antigen-coated micro-titer plate, this rinse step can be eliminated. When the rinse step is eliminated, the excess HTLV antigens are drawn off using standard suction techniques. A similar adsorption step is followed when suitable plastic beads or tubes are substituted for microtiter plates as carriers for the HTLV antigens.

After the plates are rinsed with deionized water and allowed to dry, or after the excess antigens are drawn off, an aliquot of a reagent, such as alcohol, is added to each well of the microtiter plate to dehydrate and fix the adsorbed HTLV antigens. The preferred reagent for dehydrating and fixing said HTLV antigens is absolute methanol. The plate is left to stand until all moisture is removed from the adsorbed HTLV antigens. Any remaining reagent is then removed, and the plate is allowed to air dry. Once dry, the plate is stored in a closed moisture-free container until ready to seal. After the HTLV antigens have been adsorbed to the microtiter plate and treated in the manner described above, the plates remain stable for extended periods of time of at least six months.

(3) Method of Producing Control Sera

The positive control serum is obtained from human blood serum samples which are known to contain positive levels of antibodies to HTLV antigens. For safety reasons, it may be desirable to heat inactivate any infectious agents in the blood serum samples prior to further processing. The positive human serum is

diluted in normal human serum before lyophilizing so that, upon reconstitution, the final concentration of lyophilized positive control serum will result in the desired absorbance reading by spectrophotometric methods for serum containing antibodies to HTLV antigens.

The negative control serum is obtained from normal human serum which does not contain antibodies to HTLV antigens. The undiluted negative control serum is lyophilized and, upon reconstitution, the final dilution of negative control serum is equal to the dilution of human test serum used in the assay.

(4)   Components of the Assay Kit

In a preferred embodiment, the complete assay kit of the present invention contains at least one plastic multi-well microtiter plate to which HTLV antigens have been adsorbed and treated, one vial of lyophilized human serum containing antibodies to HTLV antigens for use as a positive control, one vial of lyophilized human serum which does not contain antibodies to HTLV antigens for use as a negative control, and one vial of lyophilized enzyme-antibody conjugate, such as horse radish peroxidase-labeled or alkaline phosphatase-labeled antibody to human IgG (H&L).

The following assay components generally are not supplied with the kit but are necessary to the method of using one embodiment of the assay kit of the present invention:

1.  A serum diluent, such as 5% Bovine serum albumin or 10%

- 10 -

normal goat serum, 0.05% Tween-20 in phosphate buffered saline (PBS) (pH 7.2). Tween-20 is manufactured by Sigma Chemical Company, St. Louis, Mo.

2. A rinsing solution, such as phosphate buffered saline (PBS) containing a detergent, such as 0.05% Tween-20.

3. A substrate solution containing the substrate which will react with the specific enzyme-antibody conjugate used to measure the level of antibodies present. For example, if horse radish peroxidase is the enzyme used in the assay, an appropriate substrate solution would be as follows:

    19.6 ml  50 mM citrate buffer, pH 4.6
     0.2 ml  1% hydrogen peroxide
     0.2 ml  Orthophenylene diamine (OPD), 1%-5% in methanol

4. A stop solution for stopping the enzyme-substrate reaction, such as two normal sulfuric acid or three normal sodium hydroxide.

5. Microliter and multichannel pipettors with tips.

6. Any commercial plate reader which measures the absorbance of the reacted substrate by spectrophotometric method.

In a second embodiment of the assay kit of the present invention, a vial of lyophilized alkaline phosphatase conjugated antibody to human IgG (H&L) is supplied with the assay kit of the present invention. Accordingly, in a second embodiment, the following components also are supplied with the assay kit for use in a substrate solution:

a vial of concentrated (5x) diethanolamine (DEA) for use as a substrate buffer;

substrate tablets which contain p-nitrophenylphosphate (pNPP) or another form of concentrated p-nitrophenylphosphate.

*12*

(5) Method of Using Assay Kit

The method of using the assay kit of the present invention generally comprises the following steps:

a) Reconstituting the lyophilized positive control serum;

b) Reconstituting the lyophilized negative control serum;

c) Reconstituting and diluting the lyophilized enzyme-antibody conjugate;

d) Adding an aliquot of serum diluent to each well of an HTLV antigen-coated microtiter plate which is to contain said positive control serum, said negative control serum, or human test serum;

e) Adding an aliquot of said reconstituted positive control serum to at least one well of an HTLV antigen-coated microtiter plate;

f) Adding an aliquot of said reconstituted negative control serum to at least one well of said microtiter plate;

g) Adding an aliquot of human test serum to at least one well of said microtiter plate which contains said serum diluent;

h) Incubating said microtiter plate for such sufficient time as will permit a maximum amount of antibodies to the HTLV antigens to bind to said HTLV antigens, forming an antigen-antibody complex;

i) Rinsing each well several times, usually at least five (5) times, with a rinsing solution to remove any unbound antibodies and tapping any remaining wash drops from said plate;

j) Adding an aliquot of reconstituted and diluted enzyme-antibody conjugate to each well of said microtiter plate which contains said positive control serum, said negative control serum, or said human test serum;

k) Incubating the plate for such sufficient time as will allow said enzyme-antibody conjugate to bind to said antigen-antibody complex;

l) Rinsing said microtiter plate several times, usually at least five (5) times, with a rinsing solution and tapping any remaining wash drops from plate;

m) Adding an aliquot of a substrate solution to each well of said microtiter plate which contains said enzyme-antibody conjugate, said substrate solution being reactable with said enzyme-antibody conjugate;

n) Adding an aliquot of a stop solution to each well of said microtiter plate which contains said substrate solution;

o) Measuring the absorbance of the reacted substrate by a spectrophotometric method; and

p) Determining the presence of antibodies to HTLV antigens which may be present in said human test serum by using the absorbance measurements obtained by said spectrophotometric method.

(6)   Preferred Methods of Using Assay Kit

One preferred method of using the assay kit of the present invention generally comprises the following steps:

a)   Reconstituting a vial of lyophilized positive control serum by adding 0.10 ml of reagent quality water;

b)   Reconstituting a vial of lyophilized negative control serum by adding 0.10 ml of reagent quality water;

c)   Reconstituting a vial of lyophilized horse radish peroxidase-labeled Goat anti-human IgG conjugate by adding 0.10 ml sterile reagent quality water and, just before use, making a 1:200 dilution of said reconstituted conjugate with phosphate buffered saline containing 0.05% Tween-20;

d)   Adding 0.20 ml serum diluent to each well of an HTLV antigen-coated microtiter plate which is to contain said positive control serum, said negative control serum, or human test serum;

e)   Adding 0.10 ml of reconstituted positive control serum to at least three (3) wells of an HTLV antigen-coated microtiter plate;

f)   Adding 0.10 ml of said reconstituted negative control serum to at least three (3) wells of said microtiter plate;

g)   Adding 0.010 ml of human test serum to at least two (2) wells of said microtiter plate which contain said serum diluent;

h)   Incubating said microtiter plate at about room temperature for at least about one hour;

i) Rinsing each well several times, usually at least five (5) times, with a phosphate buffered saline solution containing 0.050% Tween-20 and tapping any remaining wash drops from said plate;

j) Adding 0.10 ml of said reconstituted and diluted horse radish peroxidase-labeled conjugate to each well of said microtiter plate which contains said positive control, said negative control, and said human test serum;

k) Incubating the plate at about room temperature for one hour;

l) Rinsing each well several times, usually at least five (5) times, with a phosphate buffered saline solution containing 0.050 % Tween-20 and tapping any remaining wash drops from plate;

m) Adding 0.10 ml of an OPD substrate solution to each well of said microtiter plate containing said positive control, said negative control, and said human test serum and incubating said plate at about room temperature for one hour;

n) Adding 0.10 ml of 2N sulfuric acid to each well of said microtiter plate which contains said substrate solution;

o) Measuring the absorbance of the reacted substrate at 490 nM on a commercial plate reader such as the Dynatech 600, manufactured by Dynatech Laboratories, Inc., 900 Slaters Lane, Alexandria, Va.; and

p)  Calculating the level of antibodies which may be present in said human test serum by the following method:

Normalize the average of all pairs of human test serum absorbances using the average of the absorbances of the wells which received negative control serum.

$$\text{Absorbance Ratio (AR)} = \frac{\text{average absorbance readings of human test serum}}{\text{average absorbance readings of negative control serum}}$$

Sera which yield an AR of $\geq$ 2.5 are presumed to contain antibodies to HTLV.  Values between 2.5 and 4.9 indicate low levels while values greater than 5.0 indicate high levels of anti-HTLV antibodies.

A second preferred method of using the assay kit of the present invention generally comprises the following steps:

a) Reconstituting a vial of lyophilized positive control serum containing antibodies to HTLV-III antigens by adding 0.10 ml of reagent quality water;

b) Reconstituting a vial of lyophilized negative control serum by adding 0.10 ml of reagent quality water;

c) Reconstituting a vial of lyophilized alkaline phosphatase conjugated antibody to human IgG (H&L) by adding 0.10 ml of reagent quality water and, just before use, making a 1:200 dilution of said reconstituted conjugate using phosphate buffered saline containing 0.05% Tween-20;

d) Adding 0.20 ml of serum diluent to each well of an HTLV-III antigen-coated microtiter plate which is to contain said positive control, said negative control, or human test serum;

e) Adding 0.01 ml of positive control serum to at least three (3) wells of said microtiter plates;

f) Adding 0.01 ml of negative control serum to at least three (3) wells of said microtiter plate;

g) Adding 0.01 ml of test serum to at least two wells of said microtiter plate;

h) Incubating said microtiter plate at about room temperature for at least about one hour;

i) Rinsing each well several times, usually at least five (5) times, with a phosphate buffered saline solution containing 0.05% Tween-20 and tapping any remaining wash drops from said plate;

j) Adding 0.10 ml of said reconstituted and diluted alkaline phosphatase conjugate to each well of said microtiter plate which contains said positive control, said negative control, and said human test serum;

k) Incubating the plate at about room temperature for at least one hour;

l) Rinsing each well several times, usually at least five (5) times, with a phosphate buffered saline solution containing 0.05% Tween-20 and tapping any remaining wash drops from said plate;

m) Making a 1:5 dilution of 5X diethanolamine (DEA) substrate buffer by adding reagent quality water which is at about room temperature;

n) Dissolving one (1) p-nitrophenylphosphate (pNPP) tablet in each 5 mls of diluted DEA to form a substrate solution;

o) Adding 0.20 ml of said substrate solution to each well of said microtiter plate containing said positive control, said negative control, and said human test serum and incubating said plate at about room temperature (18 to 22°C) for approximately about 30 minutes;

p) Adding 0.05 ml 3N sodium hydroxide to each well of said microtiter plate which contains said substrate solution;

q) Measuring the absorbance of the reacted substrate at 410nM on a commercial plate reader;

r) Calculating the level of antibodies to HTLV-III antigen which may be present in said human test serum by the method described in the first preferred method of using the assay kit of the present invention or by the following method:

Compute separately the average of the wells containing human test serum, the average of the wells containing positive control serum, and the average of the wells containing negative control serum. Add the averages of the negative and positive controls and divide by two to get the $OD_{410}$ base line value (BLV). The value of the human test serum averages must equal or exceed the base line value to be considered positive.

Example:   Negative control average equals 0.050;

Positive control average equals 0.600;

BLV equals (0.050 + 0.600) /2 = 0.325

In this example, a human test serum must yield an $OD_{410}$ of great-er than or equal to 0.325 to be considered positive.

Although only an assay kit containing an HTLV antigen-coated microtiter plate of long-term stability is described, it is contemplated that the present invention encompasses all assay kits containing an antigen-coated microtiter plate which are produced by the method described herein.

Further included within the subject invention are methods for preparing the assay kit described above which employ the technique illustrated herein.  Although only one example of an antigen-coated microtiter plate of long-term stability is given herein, it is contemplated that one skilled in the art could follow the fixation methods provided herein and produce other antigen coated microtiter plates and assay kits which exhibit the characteristics of long-term stability herein described.

Although one specific assay kit and method of producing same have been described above, it should be understood that these embodiments are described for illustrative purposes only and that numerous alterations and modifications may be practiced by those skilled in the art without departing from the spirit and scope of the invention.  Accordingly, it is the intent that the present invention not be limited to the above, but be limited only as defined in the appended claims.

CLAIMS

1. A method of producing an HTLV antigen-coated titer plate for the detection of antibodies to HTLV antigens in human sera which comprises:

a) adding an aliquot of a diluted lot of HTLV antigens to the wells of a multi-well titer plate;

b) storing said titer plate for sufficient time to permit a maximum amount of said HTLV antigens to adsorb onto said titer plate;

c) removing any unadsorbed HTLV antigens from said titer plate;

d) stabilizing said adsorbed HTLV antigens onto said plate; and

e) storing said titer plate in a moisture-free container until ready to seal said plate.

2. A method according to claim 1 wherein said aliquot of a diluted lot of HTLV antigens are adsorbed to suitable plastic beads.

3. A method according to claim 1 or 2 wherein said adsorbed HTLV antigens are stabilized by allowing said titer plate to air dry after any unadsorbed HTLV antigens have been removed from said plate.

4. A method according to claim 1, 2 or 3 wherein said adsorbed HTLV antigens are stabilized by removing any moisture from said adsorbed HTLV antigens and fixing said HTLV antigens onto said plate.

5. A method according to claim 4 wherein said moisture is removed by adding an aliquot of a reagent to said wells of said titer plate; allowing said plate to stand for sufficient time to permit said reagent to remove any moisture from said absorbed HTLV antigens; and removing any remaining drops of said reagent from said titer plate.

6. A method according to claim 5 wherein said reagent is an alcohol.

7.   A method according to claim 6 wherein said alcohol is absolute methanol.

8.   A method according to any one of claims 1 to 7 wherein said unadsorbed HTLV antigens are removed by rinsing said wells of said titer plate.

9.   A method according to any one of claims 1 to 7 wherein said unadsorbed HTLV antigens are removed from said wells of said titer plate by standard suction techniques.

10.   A method according to any one of claims 1 to 9 wherein the HTLV antigens are HTLV-III antigens.

11.   An assay kit for the detection of antibodies to HTLV antigens in human sera which comprises:

a)   a titer plate to which HTLV antigens have been adsorbed and treated;

b)   a vial of lyophilized human serum containing antibodies to HTLV antigens for use as a positive control;

c)   a vial of lyophilized human serum which does not contain antibodies to HTLV antigens for use as a negative control; and

d)   a vial of lyophilized antibody-enzyme conjugate.

12.   An assay kit according to claim 11 which also comprises

e)   a vial of substrate buffer, and

f)   a substrate.

13.   An assay kit according to claim 11 or 12 in which the HTLV antigens are HTLV-III antigens.

14.   An assay kit according to claim 11, 12 or 13 wherein the said titer plate has been produced by the method of any one of claims 1 to 10.

15.   A method for using an assay kit as claimed in any one of claims 11 to 14 which comprises the steps of:

a)   adding an aliquot of a serum diluent to each well of said titer plate which is to contain said positive control serum, said negative control serum, or human test serum;

b)   adding an aliquot of reconstituted positive control serum to at least one well of an HTLV antigen-coated titer plate which contains serum diluent;

c)   adding an aliquot of reconstituted negative control serum to at least one well of said titer plate which contains said serum diluent;

d)   adding an aliquot of human test serum to at least one well of said titer plate which contains said serum diluent;

e)   incubating said titer plate for such sufficient time as will permit a maximum amount of antibodies to the HTLV antigens to bind to said HTLV antigens, forming an antigen-antibody complex;

f)   rinsing each well of said titer plate with an appropriate rinsing solution to remove any unbound antibodies and to remove any excess of said human test serum;

g)   adding an aliquot of reconstituted and diluted anzyme-antibody conjugate to each well of said titer plate which contains aliquots of positive control serum, negative control serum, and human test serum;

h)   incubating said titer plate for such sufficient time as will allow said enzyme-antibody conjugate to bind to said antigen-antibody complex;

i)   rinsing said titer plate in an appropriate rinsing solution and removing the excess of said rinsing solution from said titer plate;

j)   adding an aliquot of a substrate solution to each well of said titer plate which contains said enzyme-antibody conjugate, said substrate solution being reactable with said enzyme-antibody conjugate;

         k)    adding an aliquot of a stop solution to each well of said titer plate containing said substrate solution to stop any enzyme-substrate reaction;

         l)    measuring the absorbance of the reacted substrate by spectrophotometric methods;

         m)    determining the presence of antibodies to HTLV antigens which may be present in said human test serum using the absorbance measurements obtained by said spectrophotometric methods.